**Europäisches Patentamt**

**European Patent Office**

**Office européen des brevets**

(19)

(11) Numéro de publication: **0 229 085**
**B1**

## (12) FASCICULE DE BREVET EUROPEEN

(45) Date de publication du fascicule du brevet: **30.05.90**

(21) Numéro de dépôt: **86903170.8**

(22) Date de dépôt: **19.06.86**

(86) Numéro de dépôt international:
**PCT/BE86/00020**

(87) Numéro de publication internationale:
**WO 87/00198 15.01.87 Gazette 87/01**

(51) Int. Cl.⁵: **C 12 N 11/08,** C 12 N 11/18,
C 12 Q 1/66, C 12 Q 1/48,
C 12 Q 1/32, G 01 N 33/53

(54) **SUPPORT POUR UTILISATION DANS LE DOSAGE PAR BIOLUMINESCENCE D'ENZYMES, DE SUBSTRATS OU D'INHIBITEURS ENZYMATIQUES.**

(30) Priorité: **26.06.85 BE 215257**

(43) Date de publication de la demande:
**22.07.87 Bulletin 87/30**

(45) Mention de la délivrance du brevet:
**30.05.90 Bulletin 90/22**

(84) Etats contractants désignés:
**AT BE CH DE FR GB IT LI LU NL SE**

(56) Documents cités:
**EP-A-0 057 600**
**EP-A-0 119 613      FR-A-2 165 832**

**Chemical Abstracts, vol. 87, no. 7, aou7t 1977, Columbus, Ohio (US); R.C. Williams: "Use of polylysine for adsorption of nucleic acids and enzymes to electron microscope specimen films", voir p. 187, abrégé 49791u**
**Chemical Abstracts, vol. 90, no. 5, janvier 1979, Columbus, Ohio (US); P.V.**

**Sundaram:"Immobilized systems: enzyme-nylon tube reactors in medical applications", voir p. 202, abrégé 35616r**

(73) Titulaire: **Remacle, Jose**
**14 Ch. des Pierres**
**B-5730 Malonne (BE)**

(72) Inventeur: **Remacle, Jose**
**14 Ch. des Pierres**
**B-5730 Malonne (BE)**

(74) Mandataire: **Van Malderen, Michel et al**
**p.a. Office van Malderen 85/042 Boulevard de la Sauvenière**
**B-4000 Liège (BE)**

(56) References cited:
**Trends in Analytical Chemistry, vol. 2, no. 11, novembre 1983, Cambridge (GB); L. Kricka et al.: "Immobilized bioluminescent enzymes", pages 244-247**

**Biotechnology & Bioengineering, vol. 27, no. 3, mars 1985, New York (US); L. Blum et al.: "Collagen strip with immobilized luciferase for ATP bioluminescence determination", pages 232-237**

**Clinical Chemistry, vol. 25, no. 4, avril 1979, Pennsylvania (US); F. Gomes et al.: "Applications of bio- and chemiluminescence in the clinical laboratory", pages 512-519**

Courier Press, Leamington Spa, England.

## Description

L'invention concerne un système de dosage constitué par une paroi sur laquelle est adsorbée au moins une luciférase qui permet en agissant de manière consécutive avec une ou plusieurs enzymes adsorbées ou non de doser par bioluminescence une série de substrats, d'enzymes du d'inhibiteurs enzymatiques. Cette invention permet de doser une série de molécules biologiques et est donc applicable dans les analyses biochimiques et dans le diagnostic médical et vétérinaire.

Certaines luciférases sont des enzymes extraites de bactéries et qui catalysent la réduction de la Flavine mononucléotide (désignée ci-après FMN) avec production concomitante de photons. Ces photons peuvent être détectés et mesurés quantitativement dans des photomètres ou par tout détecteur sensible à la lumière. Ces luciférases peuvent être associées à une NADH:FMN oxydo-réductase ou à une NADPH-FMN oxydo-réductase (NADH=Nicotinamide adenine dinucleotide sous sa forme réduite; NADPH=Nicotinamide adenine dinucleotide phosphate sous sa forme réduite). Ainsi, il est possible grâce à ces deux enzymes travaillant simultanément de doser le NADH ou le NADPH dans des zones de concentrations allant de 1 à $1000 \times 10^{-12}$ moles dans le test (Stanley, P. E. Methods in Enzymology, vol. 57, pp. 215—222, 1978; Lavi et al. J. Clin. Chem. Clin. Biochem, vol. 19, pp. 749—   , 1981).

Une autre luciférase est isolée de la luciole et permet la production de photons à partir de la coupure de l'adénosine Tri-Phosphate (ATP), en adénosine mono-phosphate (AMP) et en pyrophosphate.

Deux développements à cette méthode ont été proposés: d'une part, l'insolubilisation de ces deux enzymes sur des billes d'agarose par couplage au Bromure de Cyanogène; ensuite l'association de ces deux premières enzymes avec une troisième déshydrogénase à NADH ou NADPH qui permet de mesurer le substrat de cette dernière enzyme. Les principales réalisations sont celles de De Luca et son groupe concernant le dosage de la testostérone, du L-malate, D-glucose, 6 P-gluconate, L-lactate, de la L-alanine et du L-glutamate avec une zone de dosage minimale allant de 1,5 à $10 \times 10^{-12}$ moles (U.S. Patent N° 4,234,681 de M. A. De Luca-Mc Elroy; Weenhausen, G. and De Luca, M., Anal. Biochem., vol. 127, 380, 1982; Ford, J. and De Luca, M., Anal. Biochem., vol. 110, 43, 1981; Jallonski, E. et De Luca, M., Methods in Enzymology, vol. 57, 202, 1978). Les 12 α-hydroxy-acides biliaires peuvent aussi être mesurés (Scholemerich et al, Anal. Biochem., vol. 133, 244, 1983). La méthode peut également servir à mesurer ces déshydrogénases (Haggerty, C. et al, Anal. Biochem., vol. 88, 162, 1978). Si ce système est sensible et spécifique, il présente cependant comme désavantages d'introduire dans le système de réaction un support solide (agarose ou autre) qui provoque une certaine rétention de la lumière émise et qui est très sensible à l'agitation au moment de la mesure car l'utilisation du support poreux (agarose) nécessite la diffusion des substrats dans les mailles du gel. Une des solutions proposées à ces problèmes est de faire passer la solution en continu dans une cellule contenant le gel sur lequel soit fixées des enzymes (Kricka et al. Anal. Biochem., vol. 129, 392, 1983). Cette solution, si elle rencontre les inconvénients mentionnés ci-dessus, entraîne une complexification de la technique de dosage de l'utilisation d'un appareillage particulier.

D'autre part, récemment, V. Mookambeswaren et Sundanda (Brevet européen 0169767) ont proposé une fixation covalente de la luciférase sur un gel réalisé à partir d'albumines liées entre elles par de la glutaraldehyde.

Le document Biotechnology and Bioengineering Vol. XXVIII, mars 1985, pages 232 à 237 décrit l'immobilisation de la luciférase sur un support transparent à base de collagène ainsi que l'utilisation de celui-ci pour un dosage de l'ATP par bioluminescence.

Il s'agit toutefois de lier la luciférase par un lien covalent à une bande de collagène. Si le document mentionne une augmentation de l'activité dans le cas de l'utilisation des substrats ATP et luciférine, il y a toutefois lieu de noter que l'enzyme perd une large partie de son activité. Ce document est à rapprocher du brevet européen n° 0 169 767 (Mookambeshwaren et Sunanda).

En outre, le document (FR—A—2 165 832 décrit notamment en page 2 l'immobilisation d'enzyme sur des supports en polymères synthétiques par liaisons directes covalentes, par liaisons indirectes via un composé tiers, par des chaînages croisés de l'enzyme ou l'inclusion de celui-ci dans des réseaux polymères. Les inconvénients de ces techniques sont également cités et le brevet propose la complexation de l'enzyme avec une membrane de protéine ou polypeptide telle que le collagène.

La présente invention a pour but de remédier aux inconvénients des divers systèmes décrits ci-dessus de la manière suivante: l'invention, telle qu'elle est caractérisée dans les revendications consiste à adsorber les enzymes sur les parois du tube qui sera introduit dans le photomètre, ou sur une languette de taille et de forme appropriée à l'appareil de mesure qui peut être un simple film photographique. Cette adsorption sera effectuée par l'intermédiaire d'une couche de polylysine ou d'un copolymère de polylysine d'une autre molécule hydrophile chargée. Nous avons essayé avec succès un copolymère de polylysine et de polyphénylalanine et obtenu une fixation améliorée de la luciférase. Cette augmentation du rendement de l'adsorption s'explique par la double caractère hydrophile et hydrophobe de ce copolymère qui se fixera donc bien sur la paroi de polystyrène hydrophobe par sa partie hydrophobe en maintenant le côte hydrophile vers la solution et sur laquelle viendra s'adsorber la luciférase. Le rendement de l'adsorption est donc augmenté en utilisant un copolymère de polylysine hydrophile et d'un autre acide aminé plus hydrophobe. D'autres polymères d'acides aminés hydrophiles chargés comme la polyhistidine pourraient aussi remplacer la polylysine.

Les advantages de cette invention sont de garder la sensibilité et la spécificité du système luciférase décrit ci-dessus mais d'éviter les problèmes de rétention de lumière et de diffusion de substrat que pose l'utilisation du support poreux. Dans le cas proposé ici, l'enzyme est immédiatement en contact avec la solution de réaction. Par rapport au système de dosage dans une cellule en continu développé par Kricka et la (ci-dessus), notre invention présente l'avantage d'être plus simple, d'être immédiatement applicable aux photomètres actuellement sur le marché et de ne pas devoir tenir compte des impératifs imposés par les dosages en continu: flux constant, cellule de lecture adaptée, difficulté de production à grande échelle.

Par rapport à la proposition de Mookambeswaren et Sunanda, notre invention permet d'utiliser immédiatement le support utilisé pour le dosage (tube ou languette) et d'absorber l'enzyme directement sur ce support par l'intermédiaire d'une molécule hydrophile. Nous évitons d'une part la modification covalente de l'enzyme qui est particulièrement sensible aux agents de couplage chimique et le support utilisé est directement celui qui est utilisé pour l'appareillage de détection: tube pour un photomètre du languette pour le détecteur plan, ce qui permet la production de système de dosage prêt à l'emploi et facilite grandement l'usage industriel.

La présente invention consiste à immobiliser sur le tube ou languette qui sera introduit dans le photomètre ou devant tout autre appareil de mesure de photons, une luciférase avec, si nécessaire, une des NAD(P)H:FMN oxydo-réductases et éventuellement une ou plusieurs autres déshydrogénases ou kinases. Le tube ainsi recouvert peut servir pour le dosage du substrat des déshydrogénases ou kinases, des enzymes elles-mêmes ou d'un inhibiteur de celles-ci.

Pour ce faire, il faudra ajouter dans les proportions requises, suivant le but recherché le substrat d'une déshydrogénase (ou kinase) du NAD (ou NADP, ou ADP), du FMN (ou rien) et le substrat de la luciférase, habituellement du décanal, ces molécules seront habituellement ajoutées dans un tampon en présence de molécules stabilisatrices des enzymes. En règle générale, la molécule à tester est introduite en quantité limitante par rapport aux autres constituants du système. La lumière émise sera mesurée et habituellement intégrée pendant une période donnée afin d'augmenter lu précision du dosage.

Il existe une quantité importante de déshydrogénase à NAD ou NADP qui peuvent être utilisées consécutivement avec la NAD(P)H:FMN oxydo-réductase et la luciférase; nous en citons à titre d'exemple quelques unes ci-dessous:

   Lactose déshydrogénase
   D-glucose déshydrogénase
   β-D-galactose déshydrogénase
   glucose b-Phosphate déshydrogénase

   Mannitol déshydrogénase
   Mannitol 1 P déshydrogénase
   Sorbitol déshydrogénase
   Polyol déshydrogénase
   Pentitol déshydrogénase
   D-Xylitol déshydrogénase
   2,3-cis polyol déshydrogénase
   L-Threonic acid déshydrogénase
   Lipoyl déshydrogénase
   Lactate-déshydrogénase
   Glycoxylate déshydrogénase
   Formaldéhyde déshydrogénase
   Formate déshydrogénase
   Aldéhyde déshydrogénase
   Alcool déshydrogénase
   Acétaldéhyde déshydrogénase
   Fucose déshydrogénase
   3α-hydroxystéroïde déshydrogénase
   β-hydroxystéroïde déshydrogénase
   7α-hydroxystéroïde déshydrogénase
   3α-20β-hydroxystéroïde déshydrogénase

Nous donnons ci-après un exemple de dosage de la lactate déshydrogénase et de la testostérone en utilisant la β-hydroxystéroïde déshydrogénase. Nous avons montré que le dosage de cette enzyme pouvait être utilisé pour mesurer la concentration d'un inhibiteur de cette enzyme comme le diéthylstilbestrol (DES). La relation entre la quantité de DES et le pourcentage d'inhibition de la β-hydroxystéroïde déshydrogénase n'est pas linéaire mais le dosage de l'inhibiteur peut s'effectuer néanmoins en se référant à une courbe d'étalonnage réalisée avec une préparation étalon de DES.

Il existe aussi une quantité importante de kinases qui peuvent être utilisées consécutivement avec la luciférase à ATP. Nous en citerons quelques-unes à titre d'exemple:

   Acétate kinase
   Pyruvate kinase
   Phosphoglycéro kinase

Ces kinases peuvent être utilisées co-immobilisées avec la luciférase, ou libres en solution ou encore liées à une autre protéine dont ou veut mesurer la concentration. Par exemple, la kinase peut être mesurée tout en étant liée à un anticorps. Ceci est vrai aussi pour les déshydrogénases citées ci-dessus.

Cette liste, non exhaustive, montre la variété des substrats et enzymes qui peuvent être dosés par application du procédé. Les tubes ainsi préparés peuvent être lyophilisés et conservés pendant de longues périodes en gardant une partie importante de leur activité.

Exemple 1
Dosage du NADH et NADPH

Dans cet exemple, nous décrivons en détail l'insolubilisation de la luciférase et de la NADH:FMN oxydo-réductase. Cette insolubilisation a été optimalisée pour divers paramètres: type de support, utilisation de polylysine, concentration en poly-L-lysine, pH de fixation des ensymes, stabilisation des enzymes. En effet, l'adsorption de ces deux enzymes sert de base à l'extension de la découverte en conjonction avec d'autres déshydrogénases.

Nous avons utilisé des tubes en polystyrène spécialement conçus pour leur haut pouvoir d'adsorption (Startube, NUNC, Roskilde, Danemark). L'adsorption de la poly-L-lysine s'est faite en ajoutant par tube 0,5 ml de solution de poly-L-lysine à 80 µg/ml dans une solution de NaCl 10 mmol/l et Phosphate de Na 50 mmol/l pH 8. Les tubes sont incubés 1 heure à 20°C, avec une rotation de 5 tours par minute puis rincés 3 fois par le tampon Phosphate de Na 50 mmol/l à pH 7,5. La solution d'enzymes (0.2 ml), contenant 0,5 mg/ml de Luciférase extrait de *Vibrio-Harveyi* (Sigma, St Louis, Missouri L-1637) et 1 unité de NAD(P)H:FMN oxydo-réductase extrait de *Photobacterium fisheri* (Sigma, cat. 476.480) dissous dans un tampon phosphate de Na 50 mmol/l à pH 7.5 et contenant 2 mmol/l de dithiothréitol est alors ajoutée au tube et incubée 30 min. à 4°C sous rotation de 5 tours par min. Les tubes sont rincés 2 fois dans le tampon phosphate de Na 10 mmol/l à pH 7,5 contenant 5 mg/ml de sérum albumine bovine et 2 mmol/l dithiothréitol et conservés dans ce tampon.

Pour le dosage, le volume réactionnel de 0,5 ml comprend 2,5 µmol/l de FMN, 0,0005% de decanal, 2 mmol/l de dithiothréitol, 5 mg/ml de sérum albumine bovine, du NADH en concentrations croissantes dans le tampon Phosphate de Na 10 mmol/l à pH 7,5. L'émission maximale de lumière a été mesurée. On constate que cette émission de lumière est proportionnelle à la concentration en NADH présente dans le milieu de réaction pour une zone de concentration allant de 1 à $20.000 \times 10^{-12}$ moles dans le test. Le nombre de photons émis par sec au maximum d'intensité allant de 20 à 10.000.

Exemple 2
Dosage d'un déshydrogénase

Dans cet exemple, nous utiliserons les tubes sur lesquels ont été adsorbées la luciférase et la NADH:FMN oxydo-réductase comme décrit dans l'exemple 1 mais nous les utiliserons pour doser en solution la lactate déshydrogénase. Pour ce faire, nous ajouterons les substrats en excès de telle manière que la concentration de l'enzyme soit le facteur limitant. La solution de réaction de 0,5 ml contient 2,5 µmol/l de FMN, 0,0005% de decanal, 2 mmol/l de dithiothreitol, 5 mg/ml de sérum albumine bovine, 0,3 mmol/l de NAD$^+$ et 0,01 mol/l de L-lactate et diverses concentrations en lactate déshydrogénase. La lumière émise est mesurée et intégrée pendant 60 sec. Le dosage permet de mesurer la lactate déshydrogénase dans une zone de concentration allant de $2 \cdot 10^{-15}$ à $50 \cdot 10^{-15}$ moles dans le test.

Exemple 3
Dosage du substrat d'une déshydrogènase

Dans cet exemple, les trois enzymes: luciférase, NADH:FMN oxydo-réductase et la β-hydroxystéroïde déshydrogénase seront immobilisées sur le tube afin de réaliser le dosage de testostérone.

L'adsorption de la polylysine et des enzymes sur le tube est réalisée comme décrit dans l'exemple 1 mais la solution des enzymes comprenait 0,075 unités/ml de β-hydroxystéroïde déshydrogénase. Pour le dosage de testostérone, les tubes contenaient 0,5 ml de tampon phosphate de Na, 10 mmol/l à pH 7 et 25 µmol/l de FMN, 0,0001% de decanal, 5 mg/ml de sérum albumine bovine, 2 mMol/l de dithothreitol, 1,2 mmol/l de NAD et diverses concentrations de testostérone. L'intensité lumineuse est mesurée et intégrée pendant 60 sec. La testostérone peut être mesurée dans une zone de concentration allant de $5 \cdot 10^{-11}$ à $5 \cdot 10^{-9}$ moles dans la solution de réaction.

Exemple 4
Dosage de l'ATP par immobilisation de la luciférase à ATP

Les tubes en polystyrène à haut pouvoir d'adsorption (Startube, NUNC, Roskilde, Danemark) ont été incubés une nuit à 4°C avec une rotation de 5 tours par minute pendant 30 min en présence d'une solution de poly(lysine phénylalanine) à 80 µg/ml dans une solution tamponée à pH 8 par ou tampon phosphate 0,05 mol/l. Les tubes sont ensuite rincés 2 fois avec ce même tampon. La solution (0,2 ml) de luciférase extraite de *Photinus pyralis* (Sigma St-Louis, Missouri) contenait 40 µg/ml et des immuno-globulines G à 0,1 mg/ml dissous dans un tampon tris-acétate 0,05 mol/l, dithiothréitol 60 µmol/l, elle a été incubée 30 min, à 4°C sous rotation de 5 tours par min. Les tubes sont rincés 2 fois dans le tampon d'immobilisation des enzymes.

Pour le dosage, le volume réactionnel de 0,5 ml comprend 400 µl de tampon Tris-acétate 25 mmol/l pH 7,75; dithiothréitol 75 µmol/l, EDTA 125 µmol/l, MgCl$_2$ 6,25 mmol/l, Luciférine $7,5 \cdot 10^{-5}$ mol/l.

La réaction est démarrée avec 100 µl de solution de concentrations croissantes en ATP. L'émission de lumière est proportionnelle à la concentration en ATP dans une zone de concentration allant de 1 à $10.000 \cdot 10^{-13}$ mol/l.

Exemple 5
Dosage de la kinase

Nous avons utilisé comme exemple de dosage de kinase, l'acétate kinase qui catalyse la réaction suivante:

Acétyl-Phosphate+ADP→ATP+acétate

Cette enzyme peut être extraite de bactéries thermophiles (*Bacillus stearothermophylus*) et est particulièrement stable. Les conditions optimales du dosage de cette kinase en bioluminescence sont les suivantes:

400 µl de tampon Tris-acétate 25 mmol/l pH 7,75, EDTA 0,125 µmol/l, dithiothréitol 75 µmol/l, MgCl$_2$ 6,25 mmol/l, Luciférine $7,5 \cdot 10^{-5}$ mol/l, Acétyl Phosphate 1 mmol/l; la réaction est démarrée avec ADP $10^{-6}$ mol/l (100 µl), le dosage de l'ATP produit étant mesuré par la luciférase à ATP immobilisée sur le tube de polystyrène. Dans ces conditions des quantités aussi faibles que $10^{-17}$ et $10^{-18}$ µmole de kinase peuvent être ainsi dosées.

## Revendications

1. Procédé de dosage par bioluminescence d'enzymes, de substrats ou d'inhibiteurs enzymatiques caractérisé en ce qu'on utilise un support transparent à base d'une matière plastique synthétique traité préalablement avec une substance hydrophile chargée, pour la fixation par adsorption d'un système enzymatique contenant une ou plusieurs enzymes différentes de la luciférase et au moins une luciférase agissant de manière consécutive à cette ou ces enzymes.

2. Procédé de dosage par bioluminescence d'enzymes, de substrats ou d'inhibiteurs enzymatiques suivant la revendication 1 caractérisé en ce que la substance hydrophile contient un polymère d'acides aminés comme la polyarginine et la polyhistidine seul et/ou un de ses copolymères.

3. Procédé de dosage par bioluminescence, d'enzymes, de substrats ou d'inhibiteurs enzymatiques suivant la revendication 1, caractérisé en ce que la substance hydrophile contient la polylysine et/ou un de ses copolymères.

4. Procédé de dosage par bioluminescence d'enzymes, de substrats ou d'inhibiteurs enzymatiques suivant les revendications 1, 2 ou 3 caractérisé en ce que le système enzymatique contient la luciférase et la NADH:FMN oxydoréductase pour le dosage de NADH ou d'une déshydrogénase à NADH.

5. Procédé de dosage par bioluminescence d'enzymes, de substrats ou d'inhibiteurs enzymatiques suivant les revendications 1, 2 ou 3 caractérisé en ce que le système enzymatique contient la NADPH:FMN pour le dosage du NADH ou d'une déshydrogénase à NADPH.

6. Procédé de dosage par bioluminescence d'enzymes, de substrats ou d'inhibiteurs enzymatiques suivant les revendications 1 à 5 caractérisé en ce que le système enzymatique contient en outre une ou plusieurs déshydrogénases.

7. Procédé de dosage par bioluminescence d'enzymes, de substrats ou d'inhibiteurs enzymatiques suivant les revendications 1 à 6 caractérisé en ce que la déshydrogénase à doser est fixée à une autre protéine notamment un anticorps.

8. Procédé de dosage par bioluminescence d'enzymes, de substrats ou d'inhibiteurs enzymatiques suivant les revendications 1, 2 ou 3, caractérisé en ce que le système enzymatique contient la luciférase utilisant l'ATP comme substrat et permet le dosage de l'ATP ou d'une kinase qui produit l'ATP.

9. Procédé de dosage par bioluminescence d'enzymes, de substrats ou d'inhibiteurs enzymatiques suivant les revendications 1, 2, 3 ou 8, caractérisé en ce que le système enzymatique contient en outre une ou plusieurs kinases.

10. Procédé de dosage par bioluminescence d'enzymes, de substrats ou d'inhibiteurs enzymatiques suivant les revendications 1, 2, 3, 8 ou 9, caractérisé en ce que la kinase à doser est fixée à une autre protéine, notamment à un anticorps.

11. Support transparent à base d'une matière synthétique traité préalablement avec une substance hydrophile chargée sur lequel est fixé par adsorption un système enzymatique contenant une ou plusieurs enzymes différentes de la luciférase et au moins une luciférase agissant de manière consécutive à cette ou ces enzymes.

12. Support transparent à base d'une matière synthétique suivant la revendication 11 caractérisé en ce que la substance hydrophile contient un polymère d'acides aminés comme la polyarginine et/ou la polyhistidine et/ou un de leurs copolymères.

13. Support transparent à base d'une matière plastique synthétique suivant la revendication 11 caractérisé en ce que la substance hydrophile est la polylysine et/ou ses copolymères.

14. Support transparent suivant les revendications 11 à 13, caractérisé en ce qu'il est à base de polystyrène.

15. Utilisation du support selon l'une quelconque des revendications 11 à 14 pour le dosage des substrats NADH, NAPDH, ATP, des enzymes produisant ces substrats, à savoir les deshydrogénases et les kinases, des substrats de ces enzymes et des inhibiteurs de ces enzymes.

## Patentansprüche

1. Verfahren zur Bestimmung von Enzymen, Substraten oder enzymatischen Hemmstoffen mittels Biolumineszenz, dadurch gekennzeichnet, daß ein vorher mit einer geladenen, hydrophilen Substanz behandelter, transparenter Kunststoff-Träger verwendet wird, um ein enzymatisches System, das ein oder mehrere, von der Luciferase verschiedene Enzyme, und mindestens eine Luciferase, die nacheinander auf dieses Enzym oder diese Enzyme einwirkt, durch Adsorption zu binden.

2. Verfahren zur Bestimmung von Enzymen, Substraten oder enzymatischen Hemmstoffen mittels Biolumineszenz, gemäß Anspruch 1, dadurch gekennzeichnet, daß die hydrophile Substanz ein Aminosäuren-Polymer, wie das Polyarginin oder das Polyhistidin und/oder eines seiner Copolymere enthält.

3. Verfahren zur Bestimmung von Enzymen, Substraten oder enzymatischen Hemmstoffen mittels Biolumineszenz, gemäß Anspruch 1, dadurch gekennzeichnet, daß die hydrophile Substanz Polylysin und/oder eines seiner Copolymere enthält.

4. Verfahren zur Bestimmung von Enzymen, Substraten oder enzymatischen Hemmstoffen mittels Biolumineszenz, gemäß irgendeinem der Ansprüche 1 bis 3, dadurch gekennzeichnet, daß das enzymatische System die Luciferase und die NADH:FMN-Oxydoreduktase zur Bestimmung des NADH oder einer NADH-Dehydrogenase enthält.

5. Verfahren zur Bestimmung von Enzymen, Substraten oder enzymatischen Hemmstoffen mittels Biolumineszenz, gemäß irgendeinem der Ansprüche 1 bis 3, dadurch gekennzeichnet, daß das enzymatische System die NADPH:FMN-Oxydoreduktase zur Bestimmung des NADPH oder einer NADPH-Dehydrogenase enthält.

6. Verfahren zur Bestimmung von Enzymen, Substraten oder enzymatischen Hemmstoffen mittels Biolumineszenz, gemäß irgendeinem der Ansprüche 1 bis 5, dadurch gekennzeichnet, daß das enzymatische System außerdem eine oder mehrere Dehydrogenasen enthält.

7. Verfahren zur Bestimmung von Enzymen, Substraten oder enzymatischen Hemmstoffen mittels Biolumineszenz, gemäß irgendeinem der Ansprüche 1 bis 6, dadurch gekennzeichnet, daß die zu bestimmende Dehydrogenase an ein anderes Proteins, insbesondere einen Antikörper gebunden ist.

8. Verfahren zur Bestimmung von Enzymen, Substraten oder enzymatischen Hemmstoffen mittels Biolumineszenz, gemäß irgendeinem der Ansprüche 1 bis 3, dadurch gekennzeichnet, daß das enzymatische System die das ATP als Substrat benutzende Luciferase enthält und die Bestimmung des ATP oder einer das ATP erzeugenden Kinase ermöglicht.

9. Verfahren zur Bestimmung von Enzymen, Substraten oder enzymatischen Hemmstoffen mittels Biolumineszenz, gemäß irgendeinem der Ansprüche 1, 2, 3 oder 8, dadurch gekennzeichnet, daß das enzymatische System außerdem eine oder mehrere Kinasen enthält.

10. Verfahren zur Bestimmung von Enzymen, Substraten oder enzymatischen Hemmstoffen mittels Biolumineszenz, gemäß irgendeinem der Ansprüche 1, 2, 3, 8 oder 9, dadurch gekennzeichnet, daß die zu bestimmende Kinase an ein anderes Protein, insbesondere einen Antikörper, gebunden ist.

11. Transparenter Kunststoff-Träger, der vorher mit einer geladenen, hydrophilen Substanz behandelt wurde, und auf dem ein enzymatisches System durch Adsorption gebunden ist, das ein oder mehrere, von der Luciferase verschiedene Enzyme, und mindestens eine auf dieses Enzym oder diese Enzyme nacheinander einwirkende Luciferase enthält.

12. Transparenter Kunststoff-Träger gemäß Anspruch 11, dadurch gekennzeichnet, daß die hydrophile Substanz ein Aminosäuren-Polymer, wie das Polyarginin und/oder das Polyhistidin und/oder eines ihrer Copolymere enthält.

13. Transparenter Kunststoff-Träger gemäß Anspruch 11, dadurch gekennzeichnet, daß die hydrophile Substanz Polylysin und/oder seine Copolymeren sind.

14. Transparenter Kunststoff-Träger gemäß irgendeinem der Ansprüche 11 bis 13, dadurch gekennzeichnet, daß er aus Polystyrol besteht.

15. Verwendung des Trägers gemäß irgendeinem der Ansprüche 11 bis 14 zur Bestimmung der Substrate NADH, NAPDH, ATP, der diese Substrate erzeugenden Enzyme, nämlich der Dehydrogenasen und der Kinasen, der Substrate dieser Enzyme, und der Hemmstoffe dieser Enzyme.

## Claims

1. Bioluminescence assay method for enzymes, enzyme substrates or enzyme inhibitors, characterized in that a transparent support based on a synthetic plastic is used, the support being treated beforehand with a charged hydrophilic substance, for the binding by adsorption of an enzyme system containing one or more enzymes different from luciferase and at least one luciferase acting consecutively to this or these enzymes.

2. Bioluminescence assay method for enzymes, enzyme substrates or enzyme inhibitors according to Claim 1, characterized in that the hydrophilic substance contains a polymer of amino acids, such as polyarginine and polyhistidine alone and/or one of the copolymers thereof.

3. Bioluminescence assay method for enzymes, enzyme substrates or enzyme inhibitors according to Claim 1, characterized in that the hydrophilic substance contains polylysine and/or one of the copolymers thereof.

4. Bioluminescence assay method for enzymes, enzyme substrates or enzyme inhibitors according to Claims 1, 2 or 3, characterized in that the enzyme system contains luciferase and NADH-FMN oxidoreductase for the assay of NADH or of an NADH-dependent dehydrogenase.

5. Bioluminescence assay method for enzymes, enzyme substrates or enzyme inhibitors according to Claims 1, 2 or 3, characterized in that the enzyme system contains NADPH:FMN for the assay of NADH or of an NADPH-dependent dehydrogenase.

6. Bioluminescence assay method for enzymes, enzyme substrates or enzyme inhibitors according to Claims 1 to 5, characterized in that the enzyme system contains, in addition, one or more dehydrogenases.

7. Bioluminescence assay method for enzymes, enzyme substrates or enzyme inhibitors according to Claims 1 to 6, characterized in that the dehydrogenase to be assayed is bound to another protein, particularly an antibody.

8. Bioluminescence assay method for enzymes, enzyme substrates or enzyme inhibitors according to Claims 1, 2 or 3, characterized in that the enzyme system contains luciferase which uses ATP as a substrate, and enables ATP or a kinase which produces ATP to be assayed.

9. Bioluminescence assay method for enzymes, enzyme substrates or enzyme inhibitors according to Claims 1, 2, 3 or 8, characterized in that the enzyme system contains, in addition, one or more kinases.

10. Bioluminescence assay method for enzymes, enzyme substrates or enzyme inhibitors according to Claims 1, 2, 3, 8 or 9, characterized in that the kinase to be assayed is conjugated to another protein, in particular an antibody.

11. Transparent support based on a synthetic material and treated beforehand with a charged hydrophilic substance to which an enzyme system containing one or more enzymes different from the luciferase and at least one luciferase active consecutively to this or these enzymes is bound by adsorption.

12. Transparent support based on a synthetic material according to Claim 11, characterized in that the hydrophilic substance contains a polymer or amino acids such as polyargininie and/or polyhistidine and/or one of the copolymers thereof.

13. Transparent support based on a synthetic plastic according to Claim 11, characterized in that the hydrophilic substance to polylysine and/or copolymers thereof.

14. Transparent support according to Claims 11 to 13, characterized in that it is based on polystyrene.

15. Use of the support according to any one of Claims 11 to 14 for assaying the substrates NADH, NADPH, ATP, the enzymes which produce these substrates, namely dehydrogenases and kinases, the substrates of these enzymes and the inhibitors of these enzymes.